(19) Europäisches Patentamt
European Patent Office
Office européen des brevets

(11) **EP 3 811 934 A1**

(12) **EUROPEAN PATENT APPLICATION**
published in accordance with Art. 153(4) EPC

(43) Date of publication:
**28.04.2021 Bulletin 2021/17**

(51) Int Cl.:
*A61K 9/51* (2006.01)          *A61K 47/04* (2006.01)
*A61K 33/34* (2006.01)

(21) Application number: **18923642.5**

(22) Date of filing: **04.07.2018**

(86) International application number:
**PCT/CN2018/094390**

(87) International publication number:
**WO 2019/242044 (26.12.2019 Gazette 2019/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.06.2018 CN 201810651277**

(71) Applicant: **Suzhou Guanjie Nano Materials
Technology Co., Ltd.**
**Suzhou, Jiangsu 215000 (CN)**

(72) Inventor: **LIAN, Kun**
**Suzhou, Jiangsu 215000 (CN)**

(74) Representative: **RGTH
Patentanwälte PartGmbB
Neuer Wall 10
20354 Hamburg (DE)**

(54) **USE OF COMPOSITE NANOPARTICLE OF CARBON AND COPPER**

(57) The present invention provides use of carbon-copper composite nanoparticles in the preparation of antibacterial agents, preservatives, drugs for promoting glucose and lipid metabolism, weight-loss drugs, food additives or animal feed. The carbon-copper composite nanoparticles include a carbon shell with a porous structure and copper nanoparticles encased in the carbon shell. The present invention provides the new use of carbon-copper composite nanoparticles, which can replace the antibiotics and preservatives as antibacterial agents; improve the utilization rate of animal feed when added to animal feed; and promote glucose and lipid metabolism and reduce fat accumulation, and thus is applicable to related fields.

FIG.7

**Description**

**FIELD OF THE INVENTION**

The present invention relates to the application field of copper-containing nanoparticles, and more particularly to the use of carbon-copper composite nanoparticles.

**DESCRIPTION OF THE RELATED ART**

Copper is a trace element essential for living organisms, and is needed in animals, plants, and microorganisms. Copper plays a role in the organisms mainly by the aid of copper enzymes. It participates in the composition and activation of more than 30 enzymes, and is involved in various processes of life activities. For example, copper stimulates the immune system to fight against infection and repair injured tissues. Copper is taken in from food daily, such as some seafood and internal organs. When the daily intake of copper is too low, some copper supplements needs to be orally taken to get enough copper. In animal husbandry, copper is also added as an essential trace element in the feed of cattle, pigs, chickens and other livestock and poultry. Moreover, a large dose of copper can be added to the feed to promote growth. The main mechanism underlying the growth-promoting effect of high copper is related to enzymatic metabolism and intestinal flora.

Copper also supports the elimination of free radicals that cause serious damage to cells and plays an important role in glucose and lipid metabolism. Currently, more than 15% of the world population of 6.5 billion is overweight. There are many causes of obesity, including excessive calorie intake, genetic factors, endocrine disorders, metabolic disorders and stress. Among them, excessive energy intake is the main cause of obesity. Studies have found that there is a close relationship between insufficient lipid metabolism and copper deficiency. This is mainly because copper participates in the composition and activation of various enzymes that are closely related to the lipid metabolism, such as glutathione, peroxidase and superoxide dismutase. Therefore, the rational use of copper is effective in treating and preventing obesity. Common copper source additives include divalent copper salts such as copper sulfate, copper carbonate and copper gluconate. Such copper salts are susceptible to moisture, which affect the absorption of some vitamins, and cause environmental pollution after being excreted from the body. Therefore, stable, green and inexpensive copper source additives are urgently needed in the market.

Nanomaterials at least have one dimension in the nanoscale range in a three-dimensional space. Nanomaterials have a variety of characteristics such as volume effect, quantum size effect, and surface and interface effects. These unique properties impart nanomaterials huge application prospects. With the rapid development of nanotechnology, nanomaterials have been widely used in the area of food and animal husbandry. Due to their unique properties, nanomaterials also behavior well in antibacterial applications. Currently, nanomaterials, as new antibacterial nanomaterials, generally include inorganic metal nanomaterials, such as nano silver, nano zinc, and others, which have high antibacterial activity, broad antibacterial spectrum, and low toxicity; and antibacterial nanomaterials with photocatalytic activity, such as titanium oxide, which have relatively poor activity and usually show an antibacterial property only under UV irradiation, this greatly limits the use of such antibacterial materials.

**SUMMARY OF THE INVENTION**

To solve the above technical problems, an object of the present invention is to provide use of carbon-copper composite nanoparticles, and to further provide new use of copper source nanoparticles in the field of antibacterial application, food, and medicine.

The present invention provides use of carbon-copper composite nanoparticles in the preparation of antibacterial agents, preservatives, drugs for promoting glucose and lipid metabolism, weight-loss drugs, food additives or animal feed. The carbon-copper composite nanoparticles include a carbon shell with a porous structure and copper nanoparticles encased in the carbon shell.

Preferably, in the carbon-copper composite nanoparticles, the weight ratio of copper element to carbon element is about 1:3-3.5.

Preferably, the carbon shell is porous carbon black.

Preferably, when the carbon-copper composite nanoparticles are used as an antibacterial agent or a preservative, the dosage is 0.2 ppm or more.

Preferably, when the carbon-copper composite nanoparticles are used as animal feed, the dosage is below 800 mg/kg.

Preferably, the recommended dosage of the carbon-copper composite nanoparticles is 100-600 ppm when used as drugs for promoting glucose and lipid metabolism, weight loss drugs or food additives.

The present invention also discloses an antibacterial agent comprising the carbon-copper composite nanoparticles.

The present invention also discloses a weight loss drug comprising the carbon-copper composite nanoparticles.

The present invention also discloses an animal feed comprising the carbon-copper composite nanoparticles.

Unless otherwise specified, in the present invention, the carbon-copper composite nanoparticles (NCCC) are a material having copper as a metal core disclosed in International Publication No. WO/2007/095454 and International Application No. PCT/US2007/061862, filed on February 8, 2007 and entitled "CARBON-ENCASED METAL NANOPARTICLES AND SPONGES, METHODS OF SYNTHESIS, AND METHODS OF USE", and the preparation method is also disclosed in the patent. The carbon-copper composite nanoparticles have nanocopper evenly encased in porous carbon black, and have a balanced system of copper and cuprous oxide on the surface, which forms a unique regenerative function. This makes the additive have a low copper ion dissolution rate, and thus have a low biological toxicity, which causes less environmental pollution than other copper source additives after being excreted by metabolism.

By means of the above solution, the present invention has the following advantages.

The present invention discloses carbon-copper composite nanoparticle having integrated characteristics of antibacterial effect, growth promotion, and obesity prevention, etc, which are inorganic nanoparticles. The research in the present invention finds that the carbon-copper composite nanoparticles have excellent antibacterial effect, and have a comprehensive mechanism of action such as contact toxicity, changing the surface potential of bacteria, generating active oxygen, destroying the bacterial cell walls and direct contacting with the damaged bacterial DNA. Therefore, the carbon-copper composite nanoparticles can also be added to food or feed in place of traditional preservatives. The carbon-copper composite nanoparticles are proved to have low toxicity and no obvious accumulated toxicity in acute oral and accumulative toxicity tests in mice. In addition, it can also be used as feed, to improve the utilization rate of feed, and regulate the glucose and lipid metabolism and prevent obesity.

The above description is only a summary of the technical solutions of the present invention. To make the technical means of the present invention clearer and implementable in accordance with the disclosure of the specification, the preferred embodiments of the present invention will be described in detail with reference to the accompanying drawings.

**BRIEF DESCRIPTION OF THE DRAWINGS**

Fig. 1 is a transmission electron microscope image of carbon-copper composite nanoparticles;
Fig. 2 is a schematic diagram showing the structure of carbon-copper composite nanoparticles;
Fig. 3 shows the statistical results of the weight and food intake in different groups of mice;
Fig. 4 shows the statistical results of lean meat and fat content in different groups of mice;
Fig. 5 shows the results of oral glucose tolerance test and the statistical results of area under curve in different groups of mice;
Fig. 6 shows the statistical results of aerobic bacterium count in the feces in different groups of mice;
Fig. 7 shows the blood glucose test results in different groups of mice; and
Fig. 8 shows the test results of four items of blood lipid and the statistical results of T-CHO/HDL values in mice.

Reference numerals:

1-Carbon shell; 2-copper nanoparticles; 3-pores.

**DETAILED DESCRIPTION OF THE PREFERRED EMBODIMENTS**

The specific embodiments of the present invention will be described in further detail with reference to the accompanying drawings and specific embodiments. The following embodiments are intended to illustrate the present invention, instead of limiting the scope of the present invention.

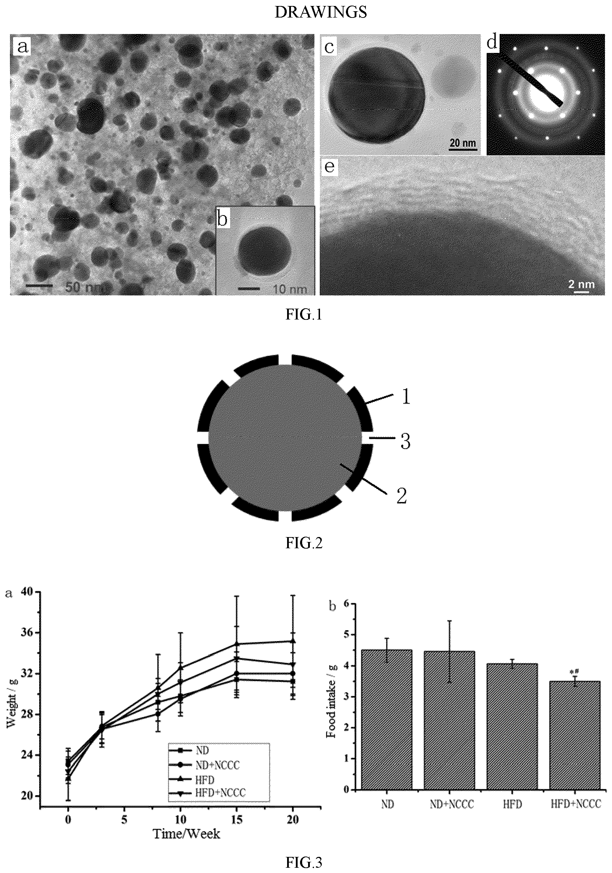

Referring to Figs. 1-2, in the following examples of the present invention, the carbon-copper composite nanoparticles (NCCC) include a carbon shell 1 with a plurality of pores 3 and copper nanoparticles 2 encased in the carbon shell 1, where the weight ratio of copper element to carbon element is 1:3. Specifically, the carbon-copper composite nanoparticles are a material having copper as a metal core disclosed in International Publication No. WO/2007/095454 and International Application No. PCT/US2007/061862, filed on February 8, 2007 and entitled "CARBON-ENCASED

METAL NANOPARTICLES AND SPONGES, METHODS OF SYNTHESIS, AND

METHODS OF USE", and the preparation method is also disclosed in the patent.

Example 1. Acute oral toxicity test in mice

The acute oral toxicity test in mice was used to determine the median lethal dose of NCCC. The specific method was as follows:

7-to-9-week-old female C57BL/6 mice were purchased from Shanghai SLAC Laboratory Animal Co., Ltd (Certificate Number: 2015000537393). 5 mice per cage were kept in a specific pathogen free (SPF) animal room at a temperature of 20°C-26°C and a relative humidity of 40%-70%, where the room was ventilated 10-20 times/h, the airflow rate was 0.1-0.2 m/s, the air cleanliness is 10000, and a 12 h-light/12 h-dark period was kept. After one week of adaptive feeding, one mouse was randomly selected to start the experiment. NCCC was ultrasonically fully dissolved in water before experiment. The experimental method was as described in the Acute Oral Toxicity Test Standard ("OECD GUIDELINE FOR TESTING OF CHEMICALS", OECD/OCDE, 2001, 12.17). A fixed dose method was employed, the initial dose was 300 mg/kg, and then the following tests were carried out based on the toxic reaction. The formal test dose was finally determined to be 1800 mg/kg. In the following two weeks, the death and toxic reactions of the mice were observed and recorded. The experimental results are shown in Table 1.

Table 1. Experimental procedures and results of acute oral toxicity in mice

| Material | Dose (mg/kg) | | Obvious toxic reaction or not | Perform the test at a next dose or not | No. | GHS |
|---|---|---|---|---|---|---|
| NCCC | Preliminary test | 300 | No obvious toxic reaction | Yes | 1 | |
| | | 1200 | No obvious toxic reaction | Yes | 2 | |
| | | 2000 | Obvious toxic reaction | No | 3 | |
| | Formal test | 2000 | Obvious toxic reaction and more than one death of mice | Yes | 4-7 | 4 |
| | Preliminary test | 1500 | No obvious toxic reaction | Yes | 8 | |
| | | 1800 | Obvious toxic reaction | No | 9 | |
| | Formal test | 1800 | Obvious toxic reaction | No | 10-13 | |

It can be seen from Table 1 that the median lethal dose of NCCC ranges from 1800 mg/kg to 2000 mg/kg, which is at level 4 according to the Globally Harmonized System of Classification and Labelling of Chemicals (GHS). The acute toxicity of NCCC in mice is mainly manifested as hypersomnia, tremor, vertical hair and hair loss. The median lethal doses of copper carbonate and copper sulfate in the acute oral toxicity experiment in mice are 320 mg/kg and 369 mg/kg, respectively. Compared with copper sulfate and copper carbonate, NCCC is less toxic, has higher safety and can be used safely over a wider range of concentrations.

Example 2: Accumulative toxicity test in mice

Each 25 of 7-to-9-week-old male and female C57BL/6 mice were purchased from Shanghai SLAC Laboratory Animal Co., Ltd. The animal feeding conditions were the same as above. After one week of adaptive feeding, the mice were randomly divided into 5 groups according to their body weight, including a control group, Group I (100 mg/kg), Group II (200 mg/kg), Group III (500 mg/kg) and Group IV (1000 mg/kg), where the ratio of female to male in each group is 1:1. A 20-day fixed dose method was used in the experiment. Each group of mice were fasted for 4 hours daily and then given a different dose of aqueous NCCC solution by gavage, and the mice in the control group were given equal volume of normal saline by gavage. The diet and the mice were weighed every week, and the food intake was calculated. The death and toxic reaction of the mice were recorded daily, and the experiment was ended after 20 days. Blood was taken from the mice, the serum was separated and then the mice were sacrificed. The internal organs were removed and weighed. The organ coefficient was calculated according to a formula. The results are shown in Tables 2-5.

$$\text{Organ coefficient (\%)} = \frac{\text{Weight of organ}}{\text{Weight of mice}} \times 100\%$$

Table 2. Accumulative toxicity test results

| Group | Dose given by gavage (mg/kg) | Number of animals | Deaths after 20 days | Survival rate (%) |
|---|---|---|---|---|
| Control group | 0 | 10 | 0 | 100 |

(continued)

| Group | Dose given by gavage (mg/kg) | Number of animals | Deaths after 20 days | Survival rate (%) |
|---|---|---|---|---|
| Group I | 100 | 10 | 0 | 100 |
| Group II | 200 | 10 | 0 | 100 |
| Group III | 500 | 10 | 0 | 100 |
| Group IV | 1000 | 10 | 0 | 100 |
| Conclusion | NCCC has no obvious accumulative toxicity. | | | |

Table 3. Test results of average body weight and feed-to-weight ratio of mice in each group ($\bar{x}\pm$SD, n=10, unit: g)

| Group | Control group | Group I | Group II | Group III | Group IV |
|---|---|---|---|---|---|
| Weight at day 0 | 18.93±1.42 | 20.50±1.06 | 20.63±3.48 | 20.13±3.26 | 20.67±3.65 |
| Weight at day 7 | 20.29±1.93 | 20.60±3.64 | 20.81±3.63 | 20.60±3.52 | 20.96±3.57 |
| Weight at day 14 | 20.89±2.22 | 21.20±3.55 | 21.50±3.76 | 20.80±3.55 | 21.42±3.55 |
| Weight at day 21 | 21.40±2.54 | 22.17±3.39 | 22.24±3.65 | 21.90±3.53 | 22.27±3.78 |
| Average body weight | 2.48±0.42 | 1.68±0.19 | 1.61±0.19* | 1.77±0.16 | 1.60±0.29* |
| Average total feed intake | 60.86 | 33.14 | 32.81 | 33.37 | 36.67 |
| Feed-to-weight ratio | 24.54 | 19.73 | 20.38 | 18.85 | 22.92 |

*P < 0.05 compared with control group

Table 4. Test results of blood biochemical indexes in the accumulate toxicity test ($\bar{x}\pm$SD, n=5)

| Indicator | Control group | Group I | Group II | Group III | Group IV |
|---|---|---|---|---|---|
| Total protein (g/L) | 51.38±0.85 | 51.06±1.15 | 49.18±1.20 | 46.02±1.62** | 43.24±1.66** |
| Albumin (g/L) | 38.75±0.76 | 38.71±0.73 | 37.07±1.165 | 33.96±1.26** | 32.11±1.93** |
| Globulin (g/L) | 12.63±0.94 | 12.35±0.52 | 12.11±1.23 | 12.06±0.82 | 11.13±2.09 |
| Albumin/Globulin ratio | 3.08±0.27 | 3.14±0.11 | 3.10±0.45 | 2.83±0.21 | 2.97±0.59 |
| Alanine aminotransferase (IU/L) | 33.76±1.26 | 33.65±1.72 | 39.28±3.99* | 40.43±1.86** | 56.90±1.43** |
| Aspartate aminotransferase (IU/L) | 97.45±2.70 | 98.78±1.42 | 108.54±7.29 | 121.62±16.36** | 137.61±4.52** |
| Urea nitrogen (mmol/L) | 7.09±0.55 | 7.25±0.35 | 7.68±0.44 | 8.67±1.02* | 9.45±0.86** |
| Creatinine (umol/L) | 9.35±0.28 | 9.59±0.28 | 9.68±0.59 | 9.96±0.76 | 11.83±1.21** |

*P < 0.05 and **p < 0.01, compared with control group

Table 5. Summary of organ coefficients of mice in each group in the accumulative toxicity experiment ($\bar{x}\pm$SD, n=10)

| Organ coefficient | Control group | Group I | Group II | Group III | Group IV |
|---|---|---|---|---|---|
| Heart coefficient | 0.55±0.06 | 0.53±0.06 | 0.56±0.06 | 0.52±0.05 | 0.57±0.04 |
| Liver coefficient | 4.20±0.17 | 4.31±0.23 | 4.35±0.27 | 4.30±0.16 | 4.52±0.16** |
| Spleen coefficient | 0.29±0.05 | 0.28±0.05 | 0.30±0.07 | 0.32±0.06 | 0.32±0.03 |
| Lung coefficient | 0.63±0.05 | 0.63±0.08 | 0.63±0.09 | 0.63±0.05 | 0.64±0.05 |
| Kidney coefficient | 1.27±0.07 | 1.29±0.03 | 1.28±0.09 | 1.29±0.05 | 1.36±0.09* |

*P < 0.05 and **p < 0.01, compared with control group

It can be seen from Table 2 that no death of mice occurs in each group, indicating that NCCC has no accumulative toxicity. According to the feed-to-weight ratio in Table 3, it can be found that NCCC can improve the feed utilization rate of mice, especially in Groups I and III. By measuring the blood biochemical indexes and organ coefficients of mice, it is found that the blood biochemical indexes of mice in Groups III and IV are abnormal, and the organ coefficients in some mice were abnormal, which indicates that the mice shows toxic reaction at a dose of 500 mg/kg or more given by gavage. There is no toxic reaction at a dose of 200 mg/kg and less given by gavage. Based on the above results, and in accordance with the industrial standards, the gavage dose of NCCC is converted into the feeding dose, so the safe feeding dose of NCCC is below 800mg/kg.

Example 3. Determination of the total bacterium count in feed

NCCC was added to the mouse feed without preservative at a dose of 600 ppm. The prepared solid medium and 0.9% sodium chloride were sterilized in an autoclave, and then allowed to stand in a water bath at 55°C for use. The feed was stood at room temperature for a week, and then ground and weighed. The feed was dissolved in physiological saline, to given three dilution gradients, including 30-fold, 300-fold, and 3000-fold dilutions respectively, and a blank control was prepared at the same time. 1 mL of the diluent was added to a blank petri dish, and then 15 mL of a medium was added, and mixed well. After the medium was solidified, it was placed upside down in a constant-temperature incubator at 37°C, and cultured for 48 h. The colony forming units were counted. The results are shown in Table 6. The results show that the total bacterium counts in the feed added with NCCC are 0 after being placed at room temperature for one week, which indicates that NCCC has an antibacterial effect in the feed and can act as a preservative.

Table 6. Test results of the total bacterium count in the feed

| Dilution factor | 30 | 300 | 3000 |
|---|---|---|---|
| Total bacterium count | 0 | 0 | 0 |

Example 4: Effect of NCCC on glucose and lipid metabolism in mice

32 4-to-5-week-old male C57BL/6 mice were purchased from Shanghai SIAC Laboratory Animal Co., Ltd. The animal feeding conditions were the same as above. After one week of adaptive feeding, the mice were randomly divided into 4 groups according to their body weight, including a normal diet group (ND), a normal diet + NCCC group (ND+NCCC), a high fat diet group (HFD), and a high fat diet + NCCC group (HFD+NCCC). In the feed added with NCCC, the dose of NCCC is 600 ppm. The food intake and weight were recorded every two weeks. The daily intake of each mouse was calculated by dividing the intake every two weeks by the number of mice and then by the number of days. Non-fasting blood glucose level was measured with a blood glucose meter every five weeks. In addition, mouse feces were collected and the aerobic bacteria in it were counted by plate count method. The experimental steps were the same as that of determination of the total bacterium count in the feed.

An oral glucose tolerance test was also conducted during the test. The specific steps were as follows. 2 g/kg glucose solution was prepared in normal saline. The glucose solution was filtered through a filter element with a pore size of 0.22 $\mu$m. The mice were fasted for 16 h and given the glucose solution at 0.1 ml/10 g. The blood glucose level was measured at 0, 30, 60, and 120 minutes, respectively. A curve was plotted by taking the time as the horizontal coordinate and the blood glucose level as the vertical coordinate. The area under curve (AUC) is calculated as follows:

$$AUC = \frac{0.5 \times (B_{g0} + B_{g30})}{2} + \frac{0.5 \times (B_{g30} + B_{g60})}{2} + \frac{1 \times (B_{g60} + B_{g120})}{2}$$

where $B_{g0}$ represents the blood glucose level/mmol·L$^{-1}$ at 0 min; $Bg_{30}$ represents the blood glucose level/mmol·L$^{-1}$ at 30 min; $B_{g60}$ represents the blood glucose level/mmol·L$^{-1}$ at 60 min; and $B_{g120}$ represents the blood glucose level/mmol·L$^{-1}$ at 120 min.

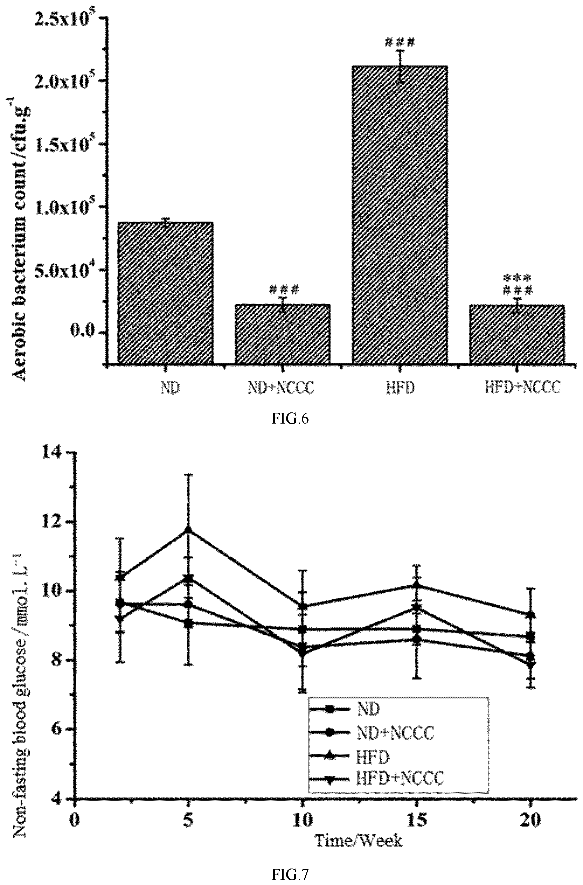

After 20 weeks, the lean meat and fat contents of mice were measured with a body composition analyzer for live small animals. Blood was collected from mice and centrifuged to obtain the serum. Four items of blood lipid, including total cholesterol (T-CHO), triglycerides (TG), high-density lipoprotein (HDL) and low-density lipoprotein (LDL), were determined according to the instructions of a relevant kit. The results are shown in Figs 3-8, where $\bar{x} \pm SD$, n=8, [#]$p < 0.05$ and [##]$p < 0.01$ compared with the ND group, and *$p < 0.05$ and **$p < 0.01$ compared with the HFD group.

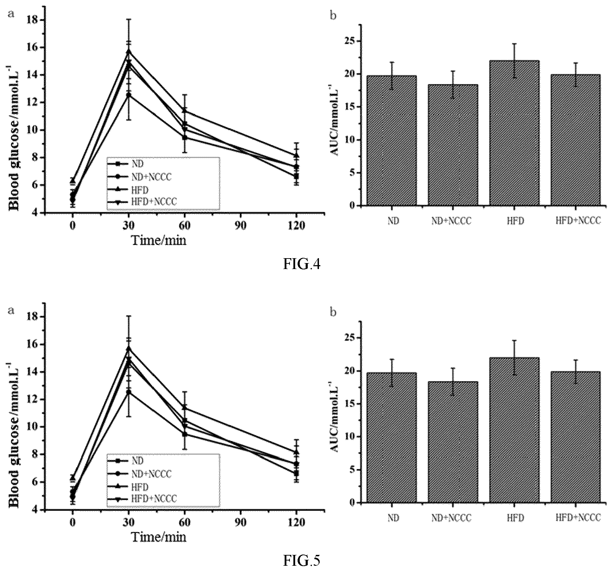

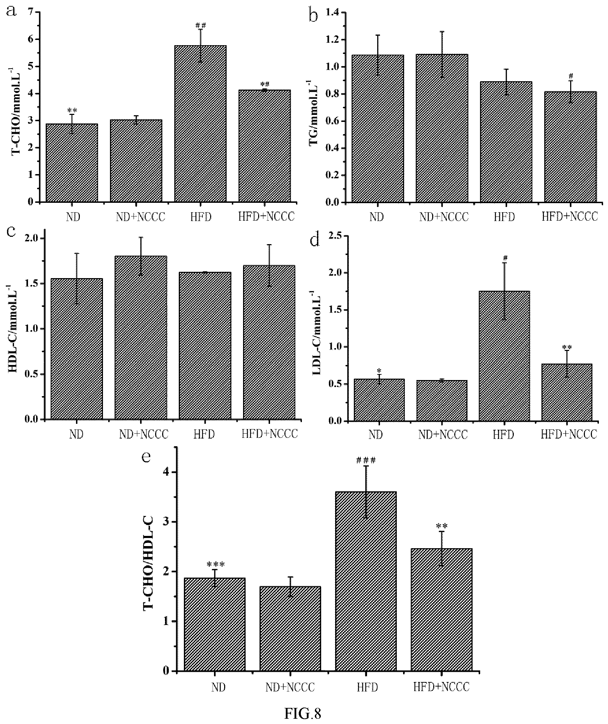

Figs. 3a and b show the statistical results of weight and food intake in different groups of mice, Figs. 4a and b show the statistical results of lean meat and fat content in different groups of mice, Figs. 5a and b show the results of oral glucose tolerance test and the statistical results of area under curve in different groups of mice respectively, and Figs. 8a, b, c, d, and e shows the serum T-CHO content, serum TG content, serum HDL content, serum LDL content, and the statistical results of T-CHO/HDL value.

The above results show that the addition of NCCC can not only reduce the animal's feed intake, but also reduce the accumulation of fat in the body, thereby reducing the weight gain of mice on a high-fat diet. Compared with the mice in the HDF group, the T-CHO/HDL level in mice of the HDF+NCCC group is significantly reduced, and there is no significant difference compared with the ND group. Within a certain range, the smaller the T-CHO/HDL value is, the stronger the body's ability to regulate blood lipids will be. It also increases the content of high-density lipoprotein and reduces the content of low-density lipoprotein. HDL can promote the elimination of cholesterol in peripheral tissues. A higher HDL content is better for the health. Elevated LDL level increases the risk of atherosclerosis and coronary heart disease, so the LDL level is preferably lower. These results indicate that NCCC can improve the ability to regulate blood lipids in mice on a high-fat diet.

The oral glucose tolerance test is a typical test of the ability to tolerate glucose in a subject. The results of this experiment show that NCCC can also improve the glucose metabolism in mice on a high-fat diet to make it close to normal. By measuring the aerobic bacterium count in the feces, it is found that NCCC can reduce the aerobic bacterium count in the feces and keep it in a stable state. The intestinal flora is closely related to glucose and lipid metabolism, and intestinal flora disorders are more likely to cause obesity. Therefore, the reason why NCCC regulates glucose and lipid metabolism may be related to this.

In summary, NCCC can promote glucose and lipid metabolism, reduce fat accumulation in the body, have an anti-obesity effect in mice on a high fat diet, and regulate the intestinal flora to stabilize it. Therefore, it can be used as a drug for promoting glucose and lipid metabolism, a weight loss drug, a food additive or animal feed.

While preferred embodiments of the present invention have been described above, the present invention is not limited thereto. It should be appreciated that some improvements and variations can be made by those skilled in the art without departing from the technical principles of the present invention, which are also contemplated to be within the scope of the present invention.

## Claims

1. A use of carbon-copper composite nanoparticle in the preparation of antibacterial agents, preservatives, drugs for promoting glucose and lipid metabolism, weight-loss drugs, food additives or animal feed, wherein the carbon-copper composite nanoparticle comprises a carbon shell with a porous structure and a copper nanoparticle encased in the carbon shell.

2. The use according to claim 1, wherein in the carbon-copper composite nanoparticle, a weight ratio of a copper element to a carbon element is about 1:3-3.5.

3. The use according to claim 1, wherein the carbon shell is a porous carbon black.

4. The use according to claim 1, wherein when the carbon-copper composite nanoparticle is used as an antibacterial agent or a preservative, a dosage of the carbon-copper composite nanoparticle to be used is at least 0.2 ppm.

5. The use according to claim 1, wherein when the carbon-copper composite nanoparticle is used as an additive in animal feed, a dosage of the carbon-copper composite nanoparticle to be used is below 800 mg/kg.

6. The use according to claim 1, wherein when the carbon-copper composite nanoparticle is used as a drug for promoting glucose and lipid metabolism, a drug for weight loss drugs or a food additive, a dosage of the carbon-copper composite nanoparticle to be used is 100 -600 ppm.

7. An antibacterial agent, comprising a carbon-copper composite nanoparticle according to any one of claims 1 to 3.

8. A weight loss drug, comprising a carbon-copper composite nanoparticle according to any one of claims 1 to 3.

9. An animal feed, comprising a carbon-copper composite nanoparticle according to any one of claims 1 to 3.

DRAWINGS

FIG.1

FIG.2

FIG.3

FIG.4

FIG.5

FIG.6

FIG.7

FIG.8

## INTERNATIONAL SEARCH REPORT

| International application No. |
|---|
| **PCT/CN2018/094390** |

**A.    CLASSIFICATION OF SUBJECT MATTER**

A61K 9/51(2006.01)i;  A61K 47/04(2006.01)i;  A61K 33/34(2006.01)i

According to International Patent Classification (IPC) or to both national classification and IPC

**B.    FIELDS SEARCHED**

Minimum documentation searched (classification system followed by classification symbols)

A61K9,A61K47,A61K33

Documentation searched other than minimum documentation to the extent that such documents are included in the fields searched

Electronic data base consulted during the international search (name of data base and, where practicable, search terms used)

CNMED; TWABS; CPRSABS; MOABS; TWMED; VEN; HKABS; CNABS: 苏州冠洁纳米抗菌涂料科技有限公司, 医药, 抗菌, 食品, 碳, 壳, 铜, 纳米, 粒子, CU, carbon, shell, nanoparticle

**C.    DOCUMENTS CONSIDERED TO BE RELEVANT**

| Category* | Citation of document, with indication, where appropriate, of the relevant passages | Relevant to claim No. |
|---|---|---|
| Y | US 9574136 B2 (LIAN, K. ET AL.) 21 February 2017 (2017-02-21) description, column 1, lines 40-44 and column 10, line 22 to column 15, line 20 | 1-9 |
| Y | CN 101362200 A (DALIAN UNIVERSITY OF TECHNOLOGY) 11 February 2009 (2009-02-11) description, page 1, paragraph 2 to page 2, paragraph 2 | 1-9 |
| A | US 2010008854 A1 (HAAM, S.J. ET AL.) 14 January 2010 (2010-01-14) entire document | 1-9 |
| A | CN 103820048 A (JIANGSU XINGHUA RUBBER BELT CO., LTD.) 28 May 2014 (2014-05-28) entire document | 1-9 |
| A | CN 102816310 A (PETROCHINA COMPANY LIMITED) 12 December 2012 (2012-12-12) entire document | 1-9 |

☐ Further documents are listed in the continuation of Box C.    ☑ See patent family annex.

|   |   |   |   |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 March 2019** | **22 March 2019** |

| Name and mailing address of the ISA/CN | Authorized officer |
|---|---|
| **State Intellectual Property Office of the P. R. China** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

| INTERNATIONAL SEARCH REPORT | International application No. |
| --- | --- |
| Information on patent family members | **PCT/CN2018/094390** |

| Patent document cited in search report | | | Publication date (day/month/year) | Patent family member(s) | | | Publication date (day/month/year) |
| --- | --- | --- | --- | --- | --- | --- | --- |
| US | 9574136 | B2 | 21 February 2017 | US | 2012021222 | A1 | 26 January 2012 |
| | | | | WO | 2007095454 | A2 | 23 August 2007 |
| | | | | US | 2017152439 | A1 | 01 June 2017 |
| | | | | WO | 2007095454 | A3 | 07 February 2008 |
| | | | | US | 2009098033 | A1 | 16 April 2009 |
| | | | | US | 8828485 | B2 | 09 September 2014 |
| | | | | US | 2014370422 | A1 | 18 December 2014 |
| CN | 101362200 | A | 11 February 2009 | | None | | |
| US | 2010008854 | A1 | 14 January 2010 | KR | 101084435 | B1 | 21 November 2011 |
| | | | | KR | 20100007665 | A | 22 January 2010 |
| | | | | US | 8916134 | B2 | 23 December 2014 |
| CN | 103820048 | A | 28 May 2014 | CN | 103820048 | B | 19 August 2015 |
| CN | 102816310 | A | 12 December 2012 | CN | 102816310 | B | 06 August 2014 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007095454 A

- US 2007061862 W

**Non-patent literature cited in the description**

- *OECD GUIDELINE FOR TESTING OF CHEMI-CALS", OECD/OCDE,* 2001, vol. 12, 17